# EUROPEAN PATENT SPECIFICATION

(11) **EP 3 863 422 B1**
(45) Date of publication and mention of the grant of the patent: **27.05.2026**
(21) Application number: 19786950.6
(22) Date of filing: 10.10.2019
(51) Int. Cl.: A23K 20/20, A23K 50/15, A61K 31/17, A61K 33/18, A61K 33/40, A61P 1/14, A61P 31/04, A61P 31/10, A61P 33/02

(54) **ANTIBIOTIC-FREE ANTIMICROBIAL FEED ADDITIVES AND ANTIMICROBIAL COMPOSITIONS**
ANTIBIOTIKAFREIE ANTIMIKROBIELLE FUTTERZUSATZSTOFFE UND ANTIMIKROBIELLE ZUSAMMENSETZUNGEN
ADDITIFS ALIMENTAIRES ANTIMICROBIENS EXEMPTS D'ANTIBIOTIQUES ET COMPOSITIONS ANTIMICROBIENNES

(30) Priority: 10.10.2018 GB 201816558
(43) Date of publication of application: 18.08.2021
(73) Proprietor: Sláinte Beoga Teoranta, Galway (IE)
(72) Inventor: O'FLAHERTY, Vincent, Galway (IE); THORN, Camilla, Galway Galway (IE); CHUI SANG, Lee, Galway Galway (IE); FRIEL, Ruairi, Galway Galway (IE); O'BRIAIN, Killian, Galway Galway (IE)
(74) Representative: FRKelly
(86) International application number: PCT/EP2019/077539
(87) International publication number: WO 2020/074672

(56) References cited:
- WO-A1-2014/177590
- WO-A1-2015/052673
- WO-A1-2016/026946
- WO-A2-2006/099359
- CN-A- 101 978 856
- JP-A- 2002 003 317
- US-A- 3 862 333
- US-A- 4 346 081
- US-A1- 2014 066 514

## Description

### TECHNICAL FIELD

This invention relates to animal feed additives and the applications of these feed additives in the improvement of the health of animals and the reduction of greenhouse gas production from animals.

This invention also relates to (i) the use of a composition comprising a source of iodide ions and a source of hydrogen peroxide, as an antimicrobial agent, where the concentration of iodide ions in use is low, (ii) a composition comprising a low relative amount of iodide ions compared with hydrogen peroxide, and its use as an antimicrobial agent, and (iii) the use of (a) a composition comprising a source of iodide ions and a source of hydrogen peroxide, in combination with (b) an antibiotic, as an antimicrobial agent. These compositions are referred to as "compositions with low concentrations", "compositions with low iodide ratios", and "antibiotic combination", respectively, throughout.

### BACKGROUND AND RELATED ART

Efficient and effective veterinary husbandry relies on healthy and disease-free animals. For example, the rearing of healthy and disease-free dairy and beef cattle helps ensure that there is sufficient provision of milk and beef for public consumption. Compared with unhealthy cows, heathy cows produce larger volumes of milk which is of better quality, and their muscle mass is gained quicker.

While antibiotics were developed for the purpose of treating infectious diseases, a discovery in the 1940s showed that administration of low levels of antibiotics to animals can allow them to act as growth promoters and enhancers of animal performance. This led to the global widespread practice of the addition of antibiotics to animal feed to stimulate growth and improve performance of the animals, with increased weight gain and improved milk yield routinely seen.

While antibiotics are an effective tool, bacteria can become resistant to them if they are misused. This presents numerous concerns not only for animal welfare but also from a human health perspective, in the transmission of antibiotic resistant bacteria to humans, and in the proliferation of cross-resistance of bacteria. Indeed, in 2006 the EU banned the use of antibiotics as a feed additive, and there has been a need to find alternative strategies.

Outside of the EU, antibiotics are routinely used for the improvement of animal performance and increased milk yield production. The ionophor monensin (sold under the trade name Rumensin^{®}) is the most widely used antibiotic feed additive; it is approved for improving efficiency in milk production, decreasing milk fat percentage and minimising body condition score during lactation.

There nevertheless exists the possibility of emergence of microorganisms resistant to this antibiotic. Studies have determined resistance to monensin from the following bacteria isolated from different animals exposed to monensin: *Clostridium aminophilum, Prevotella, Bytyrivibrio, Fibrobacter, Veilonnela, Entercoccus spp* (VKM; 2015 - The risk of development of antimicrobial resistance with the use of coccidiostats in poultry diets. Opinion of the Panel on Animal Feed of the Norwegian Scientific Committee for Food Safety, ISBN: 978-82-8259-185-0, Oslo, Norway). Of particular concern is the isolation of resistant *Enterococcus faecium* strains.

There is therefore a need for a solution to address the lack of effective alternatives to antibiotics for use in the agricultural industry, for both the mitigation of antibiotic resistance related problems and for improvement of health in animals such as cattle.

There is also a need to provide alternative ways in using antibiotics that would otherwise not be useful because the required dose of the antibiotic would be impractical (i.e. too high). Such a situation may arise where a microorganism is resistance to the antibiotic.

Another great concern in the agricultural industry is the high production of greenhouse gases, in particular methane, from the rearing of cattle for the beef and dairy industries, and more generally from ruminant animals. Methanogens such as methanogenic microorganisms residing in the rumens of these animals produce methane from the breakdown of ingested food. According to a 2006 United Nations' Food and Agricultural Organization report, the livestock sector, a large proportion of which is cows, generates 18% more greenhouse gas emissions (as measured in CO₂ equivalent) than the transport sector.

There is therefore a need to reduce the carbon footprint from animals.

Antimicrobial compositions employing reactive oxygen species such as those comprising iodide ions and hydrogen peroxide are known in the art. For example, such compositions are disclosed in WO 2014/177590. However, these compositions contain a high proportion of iodide ions compared with hydrogen peroxide and/or are used at high concentrations. US3862333 also discloses improving feed additives.

### GENERAL DEFINITIONS

The term "comprising" encompasses "including" as well as "consisting" e.g. a composition "comprising" X may consist exclusively of X or may include something additional e.g. X + Y. The term "comprising" used herein also encompasses "consisting essentially of', e.g. a composition "comprising" X may consists of X and any other components that do not materially affect the essential characteristics of the composition.

The term "about" in relation to a numerical value x is optional and means, for example, x+ 10%.

The term "antibiotic" herein is to be associated with its usual, well-known meaning. An antibiotic is something which brings about a pharmacological response, e.g. by binding to an active site. Antibiotics are generally naturally produced (or synthesized to mimic natural products). Examples of antibiotics include penicillin and amoxicillin.

The term "antimicrobial agent" means something which kills microorganisms and/or stops or slows their growth.

An antibiotic-free antimicrobial agent is any antimicrobial agent other than antibiotics.

Unless otherwise specified, the term "antimicrobial feed additive" or "feed additive" as used herein means an antimicrobial feed additive that does not encompass antibiotics.

The term "methanogenesis" means the bioproduction of methane (chemical formula = CH₄) by microorganisms e.g. bacteria or other living organisms.

The term "active ingredient" does not include excipients, fillers, food and the like, i.e. it does not include components which do not have an effect on microbes.

The term "iodide" means "iodide ions", i.e. I⁻.

The term "MIC" means minimum inhibitory concentration.

### BRIEF DESCRIPTION OF THE FIGURES

The inventions will now be described, by way of example only, with reference to the accompanying figures.
**Figure 1** shows that the feed additive of the invention results in an increase in the pH in the rumen of cows.
**Figure 2** shows that the feed additive of the invention results in a decrease in temperature in the rumen of cows.
**Figure 3** shows the decrease in cumulative methane production in cows in the presence of the feed additive of the invention
**Figure 4** shows the decrease in final percentage of methane production in cows in the presence of the feed additive of the invention
**Figure 5** shows the increase in VFA production in cows achieved in the presence of the feed additive of the invention.
**Figure 6** shows the decrease in cumulative methane production in silage-fed cows in the presence of the feed additive of the invention
**Figure 7** shows a reduction in the number of methanogens (in particular protozoa) in the rumen fluid from cows fed with the feed additive of the invention.
**Figure 8** shows that the compositions with low concentrations of the invention provide excellent release profiles.
**Figures 9 and 10** show that the low concentration and ratio compositions of the invention have a low toxicity.

The figures are explained further in the "Examples" section.

### DISCLOSURE OF THE INVENTION

This application has been drafted into sections to aid readability. However, this does not mean that each section is to be read in isolation. To the contrary, unless otherwise specified, each section is to be read with cross-referencing to the other sections i.e. taking the entire application as a whole. This means, for example, that all of the feed additives described in the "*Feed additive - ingredients and form*" section are intended to be read in combination with all of the uses of the feed additives described in the "*Uses of the feed additive*" section. Moreover, all embodiments and preferred embodiments described in the "*Feed additive*" section are intended to be combinable. No artificial separation of embodiments is intended, unless explicitly stated.

### FEED ADDITIVE

### Summary of the invention

In one aspect the present invention provides for the use of an antibiotic-free, antimicrobial feed additive in a method of improving the health of ruminant animals and reducing methane production of ruminant animals as set out in the claims.

In another aspect the present invention provides an antibiotic-free, antimicrobial feed additive, for use in a method of treating lameness in ruminant animals as set out in the claims.

A still further apsect of the present invention provides antibiotic-free, antimicrobial feed additive, for use in a method of treating acidosis in ruminant animals as set out in the claims.

A yet further aspect of the present invention provides a dosage form comprising potassium iodide and urea as the sole active ingredients, for use in a method of treating lameness in ruminant animals as set out in the claims.

A yet still further aspect of the present invention provides a dosage form comprising potassium iodide and urea as the sole active ingredients, for use in a method of treating acidosis in ruminant animals as set out in the claims.

The use of an antibiotic-free, antimicrobial feed additive in a method of improving the health of animals and reducing methane production of animals is described herein. This antimicrobial feed additive can be used to increase milk production, reduce finishing time, increase fertility, increase production of volatile fatty acids (VFAs), and reduce methane production.

The antibiotic-free antimicrobial feed additive of the invention can also be used in a method of therapy of ruminant animals comprising orally administering the feed additive, particularly where the method is for treating lameness, and/or acidosis.

The invention also relates to a dosage form comprising potassium iodide, and urea as the sole active ingredients, and the use of these dosage forms in methods of treating lameness and/or acidosis.

### Detailed description of the invention

### Feed additive - ingredients and form

The invention relates to the use of an antibiotic-free, antimicrobial feed additive in a method of improving the health of animals and reducing methane production of animals. The fact that the feed additive is antibiotic-free means that antibiotic resistance and spreading of potentially harmful residues is avoided. The antibiotic-free feed additive described herein may also be yeast-free.

The feed additive may be capable of generating a reactive oxygen species, such as sources of hypoiodite (IO⁻) and sources of hypothiocyanite ([OSCN]⁻). The feed additive may be a feed additive which is capable of generating hypoiodite (IO⁻). The feed additive may be a feed additive which is capable of generating hypothiocyanite ([OSCN]⁻).

The feed additive comprises a source of iodide ions and a source of urea. "Source of urea" means any substance able to provide urea. The source of urea may be urea or a hydrogen peroxide-urea adduct or combinations thereof. "Source of iodide ions" means anything able to provide iodides ions. Example of sources of iodide ions are iodates such as calcium iodate, iodide salts, sodium iodide (NaI), potassium iodide (KI), lithium iodide (LiI), caesium iodide (CsI), hydrogen iodide (HI), rhodium iodide (RhI₃), or other iodide ions releasing compounds, or a slow-releasing form of iodide ions such as an iodate. Combinations of such exemplary sources of iodide ions may also be used.

Potassium iodide (KI) is the source of iodide ions in the antibiotic-free, antimicrobial feed additive claimed herein. Urea is the source of urea in the antibiotic-free, antimicrobial feed additive claimed herein. As set out in claim 1, the potassium iodide is present in an amount of from 5-150 mg per gram of the feed additive and the urea is present in an amount of from 5-150 mg per gram of the feed additive, and wherein the feed additive does not contain one or more of the following vitamins: vitamin A, vitamin D and vitamin E.

The source of urea in the feed additive is present in an amount of from 5-150 mg per gram of feed additive. The source of iodide ions in the feed additive is present in an amount of from 5-150 mg per gram of feed additive. In an embodiment, the feed additive is provided as a pellet or powder, and the source of urea is present in an amount of from 5-150 mg per gram of the pellet or powder and/or the source of iodide ions is present in an amount of from 5-150 mg per gram of the pellet or powder.

The feed additive may also comprise a pharmaceutically acceptable carrier and/or diluent such as water, saline, an emulsion, a gel, a hydrogel, or a solid for pellets.

In an embodiment, the feed additive contains a source of iodide ions, specifically potassium iodideand a source of urea ions, specifically urea as the sole active ingredients. In an embodiment, the feed additive consists essentially of potassium iodide and urea. In a further embodiment, the feed additive consists of potassium iodide ions, urea, and an orally acceptable carrier.

The feed additive of the invention does not contain vitamin A, vitamin D and/or vitamin E.

In another embodiment, the feed additive of the invention is free from elements other than those explicitly disclosed herein. For example, in a preferred embodiment the feed additive of the invention does not contain chromium, cobalt, copper, manganese, selenium, magnesium and/or zinc. For example, the feed additive may contain less than 10% of chromium, cobalt, copper, manganese, selenium, magnesium and/or zinc. As a further example, the feed additive may contain less than 5% of chromium, cobalt, copper, manganese, selenium, magnesium and/or zinc. As a further example, the feed additive may contain no chromium, cobalt, copper, manganese, selenium, magnesium and/or zinc.

Such vitamins and elements are known in the art to increase milk yield in dairy cows. For example, the NRC nutrient requirements for dairy cows recommend that the following trace elements are important in increasing milk yield/production: chromium, cobalt, copper, manganese, selenium and zinc. However, surprisingly, the feed additive of the invention provides an increase in milk yield/production even without one or more of these elements present in the feed additive.

It is believed that the when the feed additive of the invention is administered to an animal it reacts in situ with an endogenous source of hydrogen peroxide to generate a reactive oxygen species. For example, when the animal is a cow, the feed additive of the invention reacts with an endogenous source of hydrogen peroxide found in the rumen of the cow. The endogenous source of hydrogen peroxide found in the rumen of the cow may be rumen microbes such as lactic acid bacteria which secrete a source of hydrogen peroxide. Various other bacteria in the rumen, such as streptococci, could act to provide an endogenous source of hydrogen peroxide. As described above, an example of a reactive oxygen species is sources of hypoiodite (IO⁻).

It has been surprisingly found that the antimicrobial feed additive of the invention does not require a source of hydrogen peroxide (because the source of hydrogen peroxide may be an endogenous source). However, the antimicrobial feed additive of the invention may optionally further comprise a source of hydrogen peroxide. The source of hydrogen peroxide may be selected from hydrogen peroxide, sodium peroxide, lithium peroxide, peroxide releasing citric acid, peroxide releasing Vitamin C, peroxide salts (e.g. barium oxide), sodium perborate, a hydrogen peroxide-urea adduct, oxygen releasing pseudo peroxides (e.g. superoxides, dioygenals, ozones, and ozonides), organic peroxides (e.g. peroxy acids, acyl halides, and aliphatic peroxides), or a peroxide -releasing percarbonate (e.g. sodium percarbonate, potassium percarbonate or a slow-releasing form of a peroxide releasing percarbonate), or permanganate in the form of a potassium salt, or combinations thereof. The source of hydrogen peroxide is preferably hydrogen peroxide.

If a source of hydrogen peroxide is present in the feed additive, the feed additive may comprise a 0.1:1 to 3:1 ratio of iodide ions to hydrogen peroxide by weight. The ratio may be 0.3:1 to 2.5:1, 0.5:1 to 2:1 or 0.5:1 to 1.5:1. Particularly preferred is a ratio of 0.5:1 to 1.5:1 by weight iodide ions to hydrogen peroxide. In another embodiment, the ratio of hydrogen peroxide to iodide ions in the feed additive is greater than 5:1, preferably greater than 10:1.

Preferably, the feed additive does not contain a peroxidase enzyme (e.g. lactoperoxidase) and/or a starch.

In an embodiment, the feed additive also comprises a cyanate compound such as potassium cyanate or thiocyanogens.

The feed additive may be provided in aqueous solution, in saline solution, as an emulsion, as a gel, as a hydrogel, as a paste, as a pellet, or as a powder. In a preferred embodiment, the feed additive is provided as a pellet. In another preferred embodiment, the feed additive is provided as a powder.

The feed additive can be provided as a pellet or powder and the urea is present in an amount of from 5-150 mg per gram of pellet or powder, and/or the potassium iodide is present in an amount of from 5-150 mg per gram of pellet or powder.

The feed additive is formulated for oral administration.

The feed additive may be adapted for use in feeding troughs and/or machines which automatically add feed additive to animal feed. The feed additive is formulated for oral administration, and may be provided as a pellet or powder and is adapted for use in feeding troughs and/or machines which automatically add feed additive to animal feed.

The feed additive may also be encapsulated, i.e. in the form of a capsule. For example, the feed additive may be encapsulated in a standard pharmaceutical capsule or a capsule used in food technology (e.g. a hard capsule such as a hard gelatin capsule or hard HPMC capsule or a soft capsule such as a soft gelatin capsule, i.e. a"softgel" capsule). The advantage of encapsulating the feed additive is that the active ingredients are kept separate from food until the feed additive is in situ. The feed additive may also be adapted to be released from encapsulated form at the pH of an animal's stomach or rumen, e.g. at a pH of from 2 to 8.

The feed additive of the invention may be formulated in a dosage form, e.g. an oral dosage form.

A particular dosage form according to the invention is a dosage form comprising potassium iodide and urea, as the sole active ingredients, as set out in the claims which may be formulated as an antibiotic-free, antimicrobial feed additive for oral administration, which may be administered in combination with food.

The feed additive of the invention can also comprise a source of thiocyanate ([SCN]⁻), which is any substance able to provide a thiocyanate ion. Exemplary sources of thiocyanate include the group consisting of: sodium thiocyanate, potassium thiocyanate, lithium thiocyanate, caesium thiocyanate, hydrogen thiocyanate, rhodium thiocyanate, and allyl isothiocyanate.

Combinations of these sources of thiocyanate may also be used. The source of thiocyanate may be present in feed additive instead of, or in addition to, the source of urea. Thus, for example, in an embodiment the feed additive may comprise a source of hydrogen peroxide (as defined herein), a source of thiocyanate, and a source of iodide ions (as defined herein). When a source of thiocyanate and a source of hydrogen peroxide are present in the feed additive, a ratio of between 0.2-5: 1 or preferably between 0.8-13: 1 of thiocyanate to the hydrogen peroxide may be used.

The feed additive which comprises potassium iodide as a source of iodide ions (as defined above in any of the amounts defined above) and urea, may comprise a source of hydrogen peroxide (as defined above in any of the amounts defined above), together with a pharmaceutically effective carrier or diluent, preferably where the feed additive does not include a peroxidase enzyme. The concentration of hydrogen peroxide may be less than 1% based on weight/volume or weight/weight, and/or the feed additive may comprise 5-5,000 mg iodide ions and a ratio of from 0.2: 1 to 3: 1 by weight of iodide ions to hydrogen peroxide.

The feed additive may also comprise, or be, any one of the "compositions with low concentrations" or "compositions with low iodide ratios" described below. Thus, purely by way of example, the feed additive may comprise a source of iodide ions and a source of hydrogen peroxide, where the ratio of hydrogen peroxide to iodide ions is greater than 5: 1.

### Subjects (animals)

The feed additive is to be used on ruminant animals. Examples of ruminants include cattle, sheep, goats, deer, giraffes, antelopes, and camels. Preferably, the animals are cows.

In an embodiment, the animal is a ruminant that is silage-fed, i.e. the ruminant has been silage fed prior to consuming the feed additive.

In an embodiment, the ruminant animal is substantially free from mastitis, i.e. if the animal is a cow, the somatic cell count (SCC) is from 100,000-200,000 somatic cells per ml of milk. In another embodiment, the ruminant animal is free from mastitis, i.e. if the animal is a cow, the somatic cell count (SCC) is less than 100,000 cells per ml of milk.

### Uses of the feed additive

The antibiotic-free, antimicrobial feed additive of the invention is used in a method of improving the health of animals and reducing methane production of animals.

The feed additive is used in a method of (i) increasing milk production, (ii) reducing finishing time, (iii) increasing fertility, (iv) increasing production of volatile fatty acids, and (v) reducing methane production.

The term "increasing milk production" may include increasing milk volume yield; and/or increasing milk solids yield. The term "increase in milk volume yield" means an increase in milk volume compared to the average volume produced by a cow without the feed additive. The term "increase in milk solid yield" means an increase in milk solid mass compared to the average mass produced by a cow without the feed additive. As shown in the examples, animals consuming the feed additive of the invention had an increased milk volume yield and solids yield compared with animals which did not take the feed additive of the invention. In an embodiment, the feed additive increases milk production in a cow which is substantially free from, or free from, mastitis.

In an embodiment, the feed additive increases milk volume yield by at least 1%. In an embodiment, the feed additive increases milk volume yield by at least 2%. In an embodiment, the feed additive increases milk volume yield by at least 5%. In an embodiment, the feed additive increases milk volume yield by at least 10%. In an embodiment, the feed additive increases milk volume yield by at least 15%. In an embodiment, the feed additive increases milk volume yield by at least 20%. In an embodiment, the feed additive increases milk volume yield by or at least 25%.

In another embodiment, the feed additive increases milk volume yield by about 1% to about 2%. In an embodiment, the feed additive increases milk volume yield by about 1% to about 5%. In an embodiment, the feed additive increases milk volume yield by about 1% to about 10%. In an embodiment, the feed additive increases milk volume yield by about 1% to about 15%. In an embodiment, the feed additive increases milk volume yield by or about 1% to about 20%. In an embodiment, the feed additive increases milk volume yield by about 1 % to about 25%.

In an embodiment, the feed additive increases milk solids yield by at least 1%. In an embodiment, the feed additive increases milk solids yield by at least 2%. In an embodiment, the feed additive increases milk solids yield by at least 5%. In an embodiment, the feed additive increases milk solids yield by at least 10%. In an embodiment, the feed additive increases milk solids yield by at least 15%. In an embodiment, the feed additive increases milk solids yield by at least 20%. In an embodiment, the feed additive increases milk solids yield by at least 25%.

In embodiment, the feed additive increases milk solid yield by about 1% to about 2%. In embodiment, the feed additive increases milk solid yield by about 1% to about 5%. In embodiment, the feed additive increases milk solid yield by about 1% to about 10%. In embodiment, the feed additive increases milk solid yield by about 1% to about 15%. In embodiment, the feed additive increases milk solid yield by or about 1% to about 20%. In embodiment, the feed additive increases milk solid yield by about 1 % to about 25%.

As shown in the examples, ruminant animals taking the feed additive had a reduced finishing time compared with animals which did not take the feed additive of the invention. "Reducing finishing time" means a reduction in the time taken to reach a final pre determined weight prior to slaughter, compared to the average finishing time of an animal without feed additive. The feed additive of the invention may reduce the finishing time by at least 1 day, at least 2 days, at least 5 days, at least 10 days, or at least 20 days.

The feed additive of the invention has also been found to increase fertility in animals and improve the success rate of conception. Specifically, ruminant animals consuming the feed additive of the invention were found to have approximately 50% fewer unsuccessful conceptions, and fewer straws were required for artificial insemination of the animals, compared with animals not consuming the feed additive of the invention.

The examples show that the feed additive of the invention results in a reduction of methane production, i.e. methane production (methanogenesis) is reduced for animals, including silage-fed animals, consuming the feed additive compared with animals that have not administered the feed additive. In an embodiment, the reduction in methane production is at least 20%.

The feed additive also provides an increase in volatile fatty acids (VFAs), as shown in the examples. Specifically, it has been found that the concentration of three VFAs (acetate, propionate and butyrate) increased when the feed additive was present, compared to when it was not. In an embodiment, the feed additive provides an increase in VFA concentration of at least 20%. These VFAs, which are produced from microbial digestion in animals such as ruminants, are the most important indicators of VFA production because they are the most rapidly metabolized. An increase in VFA is an indication of an increase in digestion in an animal, and is thus a desirable result.

The antibiotic-free, antimicrobial feed additive may be for use in a method of therapy of ruminant animals comprising administering the feed additive. It is has been observed that lameness and acidosis, in ruminant animals can be treated with the feed additive of the invention. Thus, the method of therapy of ruminant animals may include a method of treating one or more of lameness, and acidosis.

As described above, in a preferred embodiment the feed additive is formulated for oral administration. Thus, the feed additive may be administered orally. In an embodiment, the feed additive is added to food and orally administered by the animal in combination with food. In another embodiment, the feed additive is administered orally at a separate time to food.

As described above, the feed additive may be encapsulated, i.e. in the form of a capsule. Thus, in an embodiment the feed additive may be released from encapsulated form in use. Preferably, the feed additive is adapted to be released from encapsulated form at the pH of an animal's stomach or rumen, e.g. at a pH of from 2 to 8.

As described above, it is believed that the when the feed additive of the invention is administered to an animal it reacts *in situ* with an endogenous source of hydrogen peroxide to generate a reactive oxygen species. When the animal is, for example, a cow, the feed additive of the invention may react with an endogenous source of hydrogen peroxide found in the rumen of the cow. The endogenous source of hydrogen peroxide found in the rumen of the cow may be rumen microbes such as lactic acid bacteria which secrete hydrogen peroxide. Various other bacteria in the rumen, such as streptococci, could act to provide an endogenous source of hydrogen peroxide. An example of a reactive oxygen species includes sources of hypoiodite (IO⁻).

In use, the feed additive may achieve a ratio of iodide ions to hydrogen peroxide by weight in the animal's stomach/rumen of from about 0.1:1 to 3:1. The ratio may be 0.3:1 to 2.5:1, 0.5:1 to 2.0:1 or 0.5:1 to 1.5:1. Preferably, the ratio by weight of iodide ions to hydrogen peroxide is 0.5:1 to 1.5:1. In another embodiment, the feed additive may achieve a ratio of hydrogen peroxide to iodide ions by weight in the animal's stomach/rumen of greater than 5:1, preferably greater than 10:1.

For the feed additives of the invention which do not comprise a source of hydrogen peroxide, it is possible to calculate the amount of iodide ions required in the feed additive to achieve these ratios in the animal's stomach/rumen by consideration of the volume of the animal's stomach/rumen. Calculation of an amount of feed additive that would be required to achieve a certain ratio in the stomach/rumen of an animal is routine practice for a person skilled in the art.

In use, the feed additive may achieve a concentration of iodide ions in the animal's stomach/rumen of from about 0.01-500 mg/L, for example 0.01-37 mg/L or 0.03-37 mg/L. In an embodiment, the feed additive may achieve a concentration of iodide ions in the animal's stomach/rumen of from about 0.01-15 mg/L, for example from about 0.03-15 mg/L. In an embodiment, the feed additive may achieve a concentration of iodide ions in the animal's stomach/rumen of from 0.01 mg/L to less than 5 mg/L, for example from 0.03 mg/L to less than 5 mg/L. As discussed above, calculating the amount of iodide ions in the feed additive necessary to achieve this concentration in the stomach/rumen of an animal would be routine practice, e.g. it could be calculated using the known volume of the animal's stomach/rumen.

The dosage forms as set out in the claims, comprising the feed additives described herein may also be for use in a method of treating lameness in ruminant animals, or for use in a method of treating acidosis in ruminant animals, wherein optionally the method of treating involves orally administering the dosage form in combination with food.

The feed additive of the invention, or the dosage form comprising the feed additive described herein, may optionally be administered to the ruminant animal once or twice per day, optionally for 1-15 days, preferably 1-5 days.

### EXAMPLES

### Example 1 - Cattle finishing period and weight gain

### Background

Cattle reared for beef are typically housed for a final intensive finishing period where the aim is to achieve a final pre-determined weight prior to slaughter. Cattle are fed high calorie feed to boost weight gain. The amount of feed required for an animal to reach the pre-determined weight, the duration of finishing period, and the labour costs are factors that it is beneficial to minimise.

### Procedure

A cohort of 180 cattle, reared for beef production, were housed for a final intensive period of high energy feeding prior to slaughter. The cattle were split into two groups:
- Group 1 was fed once a day using a high calorie feed, without any feed additive.
- Group 2 was fed once a day using a high calorie feed, along with a feed additive of the invention (comprising urea and potassium iodide).

Cows were slaughtered when they reached the desired weight. The number of days taken to reach this weight, the deadweight of the carcass and the quality of the carcass were recorded. The deadweight is the estimated weight of saleable meat that a carcass will yield. The quality of the carcass is graded by its conformation: the overall shape and flesh coverage of the carcass. This was measured using the Beef Carcase Classification scheme: Union scale, although any conventional means would suffice. Carcasses were graded visually, upon slaughter, as either R (Good), or O (Fair). R-carcasses are superior, and earn a higher price than O-carcasses.

The total number of days to finish each group of cattle is the sum of each row for (Days to Reach Finishing Weight x Number of Cattle).

The total deadweight of each group of cattle is the sum of each row for (Deadweight x Number of Cattle).

### Results

### Group 1 - Control

| **Table 1: Time to reach finish weight, total deadweight, and quality of carcasses for cattle without feed additive during finishing period** | | | | |
|---|---|---|---|---|
| **Days to Reach Finishing Weight** | **Number of Cattle Slaughtered** | **Deadweight (kg)** | **# R-carcasses** | **# O-carcasses** |
| 98 | 24 | 330 | 8 | 16 |
| 106 | 15 | 315 | 2 | 13 |
| 111 | 15 | 331 | 1 | 14 |
| 116 | 11 | 323 | 2 | 9 |
| 119 | 9 | 307 | 1 | 8 |
| 130 | 5 | 343 | 1 | 4 |
| 138 | 6 | 282 | 0 | 6 |
| 144 | 3 | 279 | 1 | 2 |
| 152 | 1 | 246 | 1 | 0 |
| Number of cattle = 89 | | | | |

| | | | | |
|---|---|---|---|---|
| Total number of days for Group 1 to reach finishing weight = 10016 Average number of days for a cow in Group 1 to reach finishing weight = 112.54 Total deadweight of Group 1 = 28416 kg Average deadweight of a cow in Group 1 = 319.28 kg Total R-carcasses = 17 % R-carcasses = 19.10% Total O-carcasses = 72 % O-carcasses = 80.90% | | | | |

### Group 2 - with feed additive

| **Table 2: Time to reach finish weight, total deadweight, and quality of carcasses for cattle with feed additive during finishing period** | | | | |
|---|---|---|---|---|
| **Days to Reach Finishing Weight** | **Number of Cattle Slaughtered** | **Deadweight (kg)** | **# R-carcasses** | **# O-carcasses** |
| 59 | 15 | 324 | 3 | 12 |
| 83 | 7 | 330 | 4 | 3 |
| 89 | 19 | 322 | 7 | 12 |
| 97 | 14 | 323 | 7 | 7 |
| 103 | 15 | 325 | 4 | 11 |
| 109 | 16 | 315 | 5 | 11 |
| 120 | 5 | 322 | 0 | 5 |
| Number of cattle = 91 | | | | |

| | | | | |
|---|---|---|---|---|
| Total number of days for Group 2 to reach finishing weight = 8408 Average number of days for a cow in Group 2 to reach finishing weight = 92.35 Total deadweight of Group 2 = 29335 kg Average deadweight of a cow in Group 2 = 322.36 kg Total R-carcasses = 30 % R-carcasses = 32.97% Total O-carcasses = 61 % O-carcasses = 67.03% | | | | |

### Discussion

The data from Tables 1 and 2 show that the average number of days to reach the finishing weight was significantly lower in the cattle that were administered the feed additive than those that were not administered a feed additive. An average reduction in the time to reduce the finishing weight of around 20 days (which equates to an 18% decrease) was seen. This has multiple benefits to the farmer e.g. on saving on the cost of feed, labour and housing.

The data from Tables 1 and 2 also show that there is a significantly lower variation in the deadweight of the carcasses from the cattle administered the feed additive, compared to those that were not. All carcasses in Group 2 had a deadweight of between 315 and 330 kg, whereas group 1 had a range of carcass weights between 246 and 343 kg.

The quality of the carcasses from the cattle that were administered the feed additive were also, in general, superior. A 13.87% increase in the number of R-carcasses was shown for the cows that were administered the feed additive, in comparison to those that were not.

### Example 2 - Milk volume yield and milk solids content

### Procedure

A cohort of 180 cows was split into two groups. Some of the cows were free-from mastitis, some were substantially free from mastitis and some had mastitis. Both groups were monitored for their milk volume yield and the milk solid content of their milk.

### Study A

This study took place over an 8-month period from February to September. The groups were fed as follows:
• Group 1 was fed a normal feeding routine, without any feed additive.
• Group 2 was fed a normal feeding routine, that included the feed additive of the invention (comprising urea and potassium iodide)

**Table 3: Daily milk volume yield, and daily milk solid content in cows fed with and without the feed additive**

| **Average daily milk yield (L)** | | | **Average daily milk solid content (kg)** | | |
|---|---|---|---|---|---|
| | Group 1 | Group 2 | | Group 1 | Group 2 |
| Feb | 14.7 | 14.9 | Feb | 1.14 | 1.15 |
| Mar | 15.1 | 16.4 | Mar | 1.17 | 1.28 |
| Apr | 14.9 | 18.4 | Apr | 1.17 | 1.42 |
| May | 17.5 | 20.3 | May | 1.36 | 1.59 |
| Jun | 17.5 | 19.7 | Jun | 1.36 | 1.56 |
| Jul | 16.6 | 17.7 | Jul | 1.3 | 1.42 |
| Aug | 12.7 | 15.4 | Aug | 1 | 1.23 |
| Sep | 11.9 | 11.9 | Sep | 0.95 | 0.98 |

The data in Table 3 shows consistently higher milk volume yield and milk solid content for Group 2 compared to Group 1 over the course of the study. The feed additive resulted in a higher milk volume yield and milk solid content for all cows, i.e. regardless of whether or not the cows had mastitis.

### Study B

This study took place over a 10 month period from January to October. The groups were fed as follows:
- Group 1 was fed a normal feeding routine, without any feed additive.
- Group 2 was fed a normal feeding routine that included the feed additive for the months of January to April, and September to October. For the months of May to August, Group 2 was fed a normal feeding routine, without any feed additive.

**Table 4: Daily milk volume yield, and daily milk solid content in cows fed with feed additive over two time periods with a break, and cows fed without feed additive for the duration**

| **Average daily milk yield (L)** | | | **Average daily milk solid content (kg)** | | |
|---|---|---|---|---|---|
| | Group 1 | Group 2 | | Group 1 | Group 2 |
| Jan | 0 | 0 | Jan | 0 | 0 |
| Feb | 8.9 | 12 | Feb | 0.74 | 0.98 |
| Mar | 16.1 | 16.5 | Mar | 1.23 | 1.22 |
| Apr | 16.4 | 19.5 | Apr | 1.2 | 1.38 |
| May | 21.4 | 21 | May | 1.58 | 1.54 |
| Jun | 19.5 | 18.6 | Jun | 1.43 | 1.37 |
| Jul | 17.8 | 17.4 | Jul | 1.32 | 1.29 |
| Aug | 16.2 | 17.5 | Aug | 1.24 | 1.35 |
| Sep | 14 | 16.2 | Sep | 1.12 | 1.31 |
| Oct | 9.8 | 11.3 | Oct | 0.81 | 0.99 |

The data in Table 4 demonstrates that removing the feed additive from the cattle previously fed with it returns the animals to around normal milk volume yield and milk solid content. When these animals are then re-administered the feed additive following 4 months without it, the stark increase in milk volume yield and milk solid content is seen once again.

This demonstrates that the feed additive is effective on a short timescale, and is particularly effective when administered regularly (e.g. every day). This is also advantageous as it demonstrates that the feed additive is not having potentially deleterious effects on the animal that could be caused by longer lasting effects.

This data also demonstrates that the feed additive is still effective if the animal has previously been administered it, and that the animal does not develop a resistance to the feed additive over time, as might be expected with antibiotic-containing feed additives.

### Example 3 - Other general health benefits

### Decrease in lameness

Lameness is a common and costly illness of, for example, dairy cows, affecting up to 35% of the herd annually. This causes many economic issues to the farmer, including reduced milk yield by up to 350kg per lactation, reduced fertility, increased risk of secondary disease, loss of condition and increased risk of culling.

Approximately 33% of all cases of lameness are classified as white line disease or laminitis.

Treatment of cows with the feed additive of the invention demonstrated (i) a marked reduction in the incidence of lameness in the herd of 25-30% and (ii) a reduction in instances of lameness due to laminitis or white line disease. The cure rates of cows with laminitis or white line disease was found to be 90-95%.

### Increased fertility rate (illustrative to the claimed invention)

Due to improved metabolic performance and attainment and maintenance of positive energy balance an improved rate of fertility was seen in cows consuming the feed additive of the invention. The number of unsuccessful conceptions was 50% fewer, i.e. in trial herds an approximate rate of 15% of the herd shall not conceive and deliver a calf; when the feed additive of the invention was added to the diet this figure dropped to 8%, yielding economic gains.

Additionally, the success rate of conception was increased in cows receiving the feed additive of the invention. Artificial insemination is a common means of controlling the reproductive cycle of a cow, with straws of frozen semen introduced into the reproductive tract. This will not have a 100% success rate and so multiple straws are used until a successful conception is seen. In a typical 1OO-cow herd receiving the feed additive of the invention 70-80 fewer straws were required. This has an obvious economic cost due to reduced purchase of straws, but also allows for a more synchronised birthing in the herd, which shall allow for a greater number of calves born to in turn become pregnant by year 2, delivering greater productivity to the farm.

### Impact on somatic cell count and incidence of mastitis (illustrative to the claimed invention)

Mastitis is an inflammatory disease caused by a bacterial infection of the cow udder. Mastitis may present as either sub-clinical (lower yields and quality of milk, but no obvious clinical signs) or clinical (clumped, discoloured milk, udder swelling, tenderness, animal discomfort). Typically, mastitis is diagnosed through a quantification of somatic cells in the milk. A low somatic cell count (SCC) is indicative of healthy milk, with rising levels showing sub-clinical and clinical infection. Mastitis is the major economic cost to dairy farmers, with each case of mastitis estimated at €265/cow, and collectively causes losses of approximately €2 billion per annum to the European dairy sector and $2 billion losses to the US dairy sector. Mastitis affects milk production in a number of ways: milk yield is lowered and the quality of this milk is reduced - collectively as producers are paid based upon milk quality, this means producers receive less money for less milk volumes produced. Additional costs include treatment costs (medicine and veterinarian time) and should treatment fail, culling and replacement of the cow (typically at a cost of ~€l, 500/cow).

Due to improved metabolic performance and attainment and maintenance of positive energy balance improved milk quality and reduced incidence of mastitis (>50%) was seen in cows consuming the feed additive of the invention. The somatic cell count was also reduced (by 33%) in the cows receiving the feed additive of the invention.

### Effect on ringworm (illustrative to the claimed invention)

Ringworm is a fungal infection. This manifests itself through hair loss, followed weeks later by the formation of a dry grey crust and circular lesions. Spread from animal-to-animal is quite rapid and can quickly infect a large proportion of the herd.

A dramatic effect on the treatment and cure of ringworm in cattle infected has been seen.

Following 3 weeks of consumption of the feed additive of the invention, a cure rate of 90% was seen.

### Effect on acidosis

Rumen acidosis is a metabolic disease of cattle. It is thought to occur when the pH of the rumen falls below approximately 5.5. This has two consequences for the cattle: (i) the rumen stops moving and becomes atonic, which depresses appetite and production, and (ii) the change in acidity changes the rumen flora, with acid-producing bacteria taking over. More acid is produced, which makes acidosis worse. The increased acid is absorbed through the rumen wall, causing metabolic acidosis, which in severe cases can lead to shock and death.

| **Milk production** | | | | |
|---|---|---|---|---|
| | **Unit** | **2018** | **2019** | **2020** |
| In the morning | **Stk** | 54.8 | 53.5 | 51.3 |
| Milk Quantity | **Kg** | 12.024 | 11.291 | 11.273 |
| Fat | **%** | 4.1 | 4.2 | 4.3 |
| Ew | **%** | 3.6 | 3.6 | 3.6 |
| F/Ew | **Kg** | 930 | 888 | 895 |
| Milk production value | | 147 | 149 | 149 |

The lactating cows get an upgraded forage ration and each animal get individually a concentrated feed supplement on a transponder station. The feed additive of the invention (comprising urea and hydrogen peroxide) was mixed into the concentrated feed (coarse grain) making up 10%. This mixture was administered via a mineral feed dispenser to the group "Feed additive group" at a rate of 100 grams per day. The control group did not receive this concentrate.

The end of the trial was set by the duration of the data delivery of the pH sensors. The data collection took place in the first 60 days of lactation as rumen acidosis is most likely to occur during this period.

| | **Control group** | **Feed additive group** |
|---|---|---|
| Grassilage | 6 | 6 |
| Maizesilage | 6 | 6 |
| Hay | 2.7 | 2.7 |
| Cereal grain | 1.1 | 1.1 |
| Maize grain | 0.5 | 0.5 |
| Rindastar 39 XP ATG | 1.1 | 1.1 |
| Mineralfeed | 0.15 | 0.15 |
| Rumen Proof | 0 | 0.1 |
| Kuhkorn Vital Plus | Transponder - performance orientated | |
| Kuhkorn Select Megalac | Transponder - performance orientated | |

### • Milk Protein:

| **Group** | **Protein %** |
|---|---|
| Control | 3.3 |
| Feed additive | 3.43 |

The table above shows that the percentage of protein was increased in the cattle which administered the feed additive of the invention. Thus, the milk quality in these cattle is improved.

### • Statistical data evaluation of the results of the rumen sensors - material, method and results

For the evaluation, sensor data (pH, temperature, activity) of the company SmaxTec were used. The data was taken of 14 cows which were divided into 2 groups, a control and feed additive group (control = 0, feed additive group = 1). Due to the set trial duration the cows are varying in their lactation stages. In this trial data was analyzed from cows two weeks out from calving until week 18 of lactation. The raw data was tested for any deviations within the overall material, taking into account additional measurement aspects for the individual parameters. In accordance with the rules for outlier evaluation, three analysis had to be taken out, which are (animal 16) for pH data, (animal 71) for activity data and (animals 16, 34, 46, 56 and 71) for the temperature analysis.

For the statistical evaluation a simple multiple linear model (GLM, Statgrapics XVII) was used and a test for normal distribution took place. This tests the dependent variables (pH, temperature, activity) in connection with the classes (group, lactation week) and the possible interaction between them.

The following results could be derived:

### (i) pH:

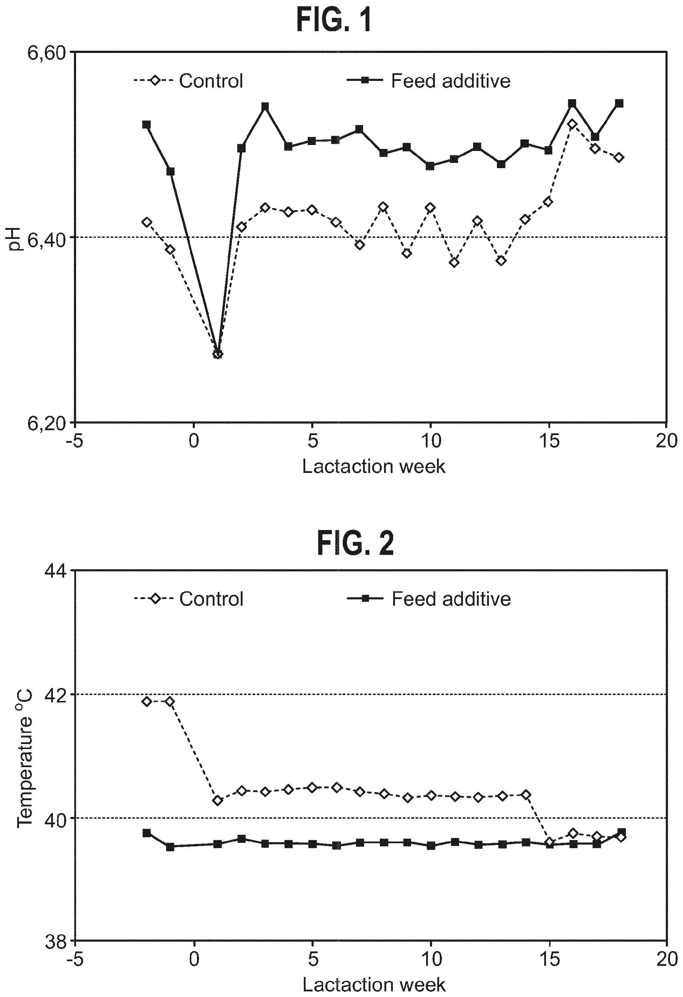

As shown in Figure 1, the pH is significantly higher when the feed additive of the invention is used compared with the control group. In relation to the lactation stages the results show that the lactation week 1 differs significantly from all other weeks. The interactions don't show any significance.

### (ii) Temperatures (°C)

As shown in Figure 2, the raw data of the temperature were taken at water intake and the findings show a highly significant difference between control and feed additive group. The higher temperatures in the untreated animals (i.e. the control group) compared with those taking the feed additive of the invention suggests a very rapid or imbalanced fermentation which would lead to the generation or accumulation of acidic products in a way that could promote acidosis. Neither the lactation week nor the interaction show any significance.

### • Summary of results

The use of the feed additive of the invention resulted in a highly significant change in the pH (an increase, i.e. less acidic) and a reduced temperature in the Rumen. The higher pH in the rumen had no detrimental effect on the performance or milk solids.

The feed additive of the invention had an effect on the rumen flora in this trial as it modulates rumen pH and temperature, which are parameters strongly influenced by microbial activity. Based on these results it is expected that the feed additive of the invention can be used to treat acidosis in animals such as cattle.

### Example 4 - Effect on of the feed additive on methanogenic activity and VFAs

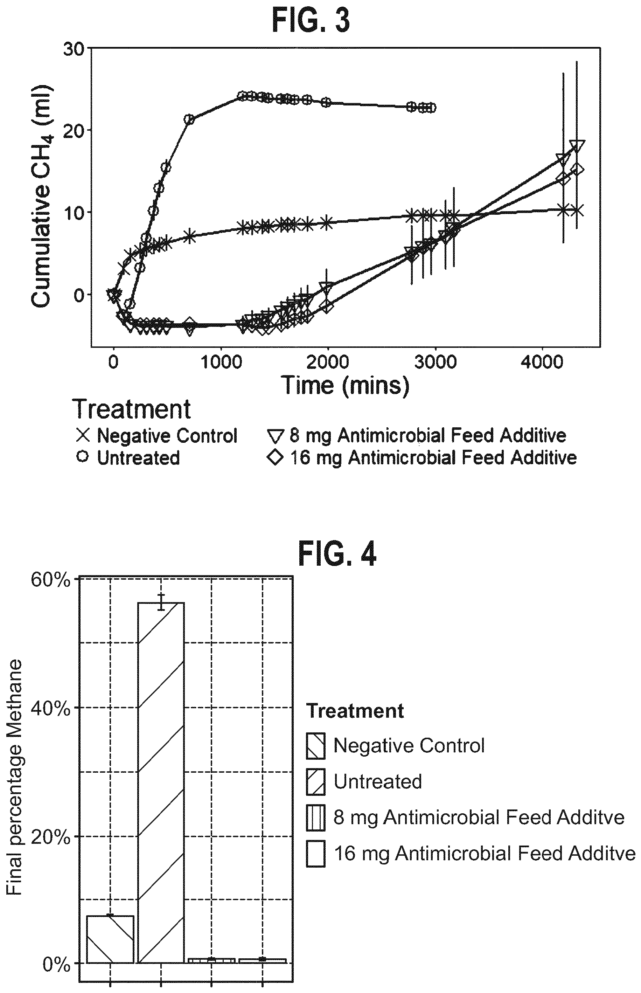

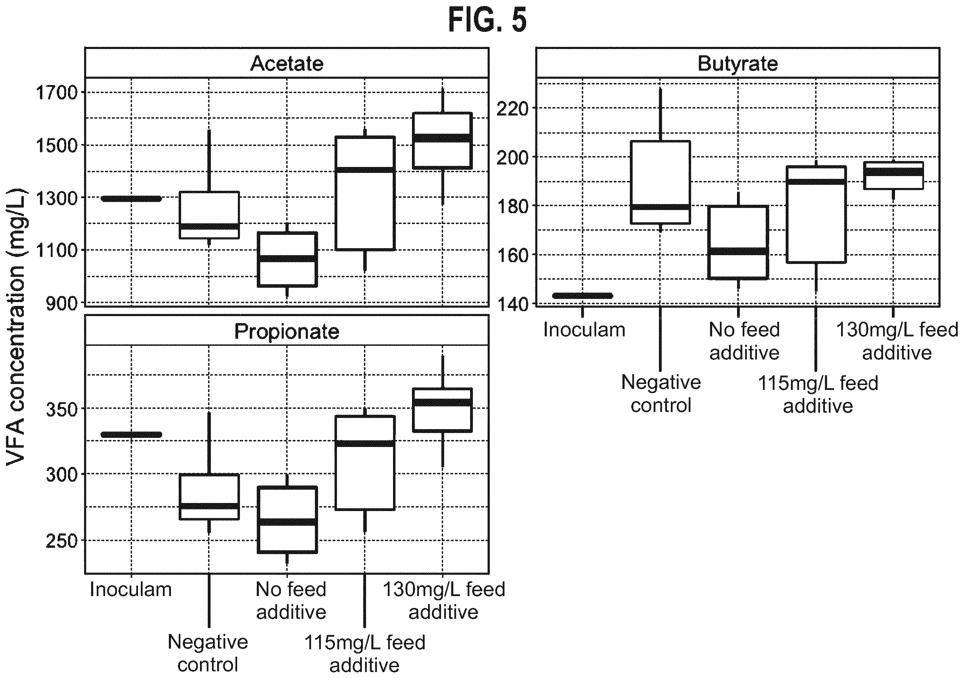

Example 4 (results shown below and in Figures 3-6), is an in vitro example which illustrates the reduction in methane production and increase in VFA production achieved in the presence of the feed additive of the invention. The feed additive used in this example contains a source of hydrogen peroxide. However, as described above, the source of hydrogen peroxide is not an essential part of the feed additive. When the feed additive of the invention is administered to an animal it reacts in situ with an endogenous source of hydrogen peroxide to generate a reactive oxygen species. Thus, the source of hydrogen peroxide in the feed additive in this example is only present to compensate the fact that this example is an in vitro example. The reduction in methane production shown in this example would also be achieved in vivo with a feed additive that does not contain a source of hydrogen peroxide.

### Method (a)

### Sample collection

Rumen content was collected from cows immediately following slaughter from an abattoir, and stored in a vacuum flask at room temperature with frequent ventilation of biogas. Within 24 hours of slaughter, the rumen content was filtered using a 200 mM membrane to remove large organic matter, and retain protozoa. The filtrate was used to inoculate media in batch assays.

### Batch assays

Batch assays were carried out using 110 ml anaerobic vials in triplicate. Each vial contained nutrient buffers and 6 ml inoculum, and the feed additive comprising a peroxide -urea adduct and potassium iodide in a 3: 1 ratio (or, for controls, more nutrient buffer) was added, to a final volume of 45 mL.

The headspace gas in the vials is evacuated, and replaced with H 2 and CO2 gas, as substrates for methanogenesis. For a negative control, one vial is instead filled with N2 and CO2 gas, and not inoculated. Vials were incubated at 39 °C for 24-48 hours with shaking at 100 rpm.

### Variables measured

- Changes in headspace pressure at regular intervals (electronic transducer measured in mV, converted to mL) for methane production.
- % methane in headspace at final timepoint (from methane Gas Chromatography).
- 2 mL liquid samples taken for Volatile Fatty Acid (VFA) analysis (analysed by GC)

### Results

The results are shown in Table 5 below and in Figures 3-5.

**Table 5: Specific methanogenic activity at varying concentrations of feed additive**

| **Treatment** | **Specific methanogenic activity (ml CH₄/g VS / day)** |
|---|---|
| Negative control | N/A |
| No antimicrobial agent | 96.09085 |
| 80 mg/L feed additive | 77.17748 |
| 100 mg/L feed additive | 85.54866 |
| 115 mg/L feed additive | 63.92205 |
| 130 mg/L feed additive | 70.64753 |

### Method (b)

Fresh rumen fluid was filtered at 200 mM to remove particulate organic matter while keeping protozoa, and mixed with an anaerobic buffer. Additionally, 400 mg of dried silage was placed in each vial. Non-fed controls were included to consider any background methane production.

The feed additive (comprising a hydrogen peroxide-urea adduct and potassium iodide in a 3:1 ratio) was added to half the'fed' vials to determine the effect on rates of methanogenesis, and vials place shaking at 37°C
Vial headspace for all samples consisted of N₂CO₂ (ie no H₂ substrate for hydrogenotrophic methanogens meaning silage needed to be broken down in order to provide substrates for methane production).

Thus, methanogenesis using silage substrate was measured via increasing pressure (using a pressure transducer) which was equated to cumulative CH₄ production.

At the end of the experiment (when all headspace gas was consumed) a gas sample was removed from each vial and the actual percentage methane was measured using gas chromatography .

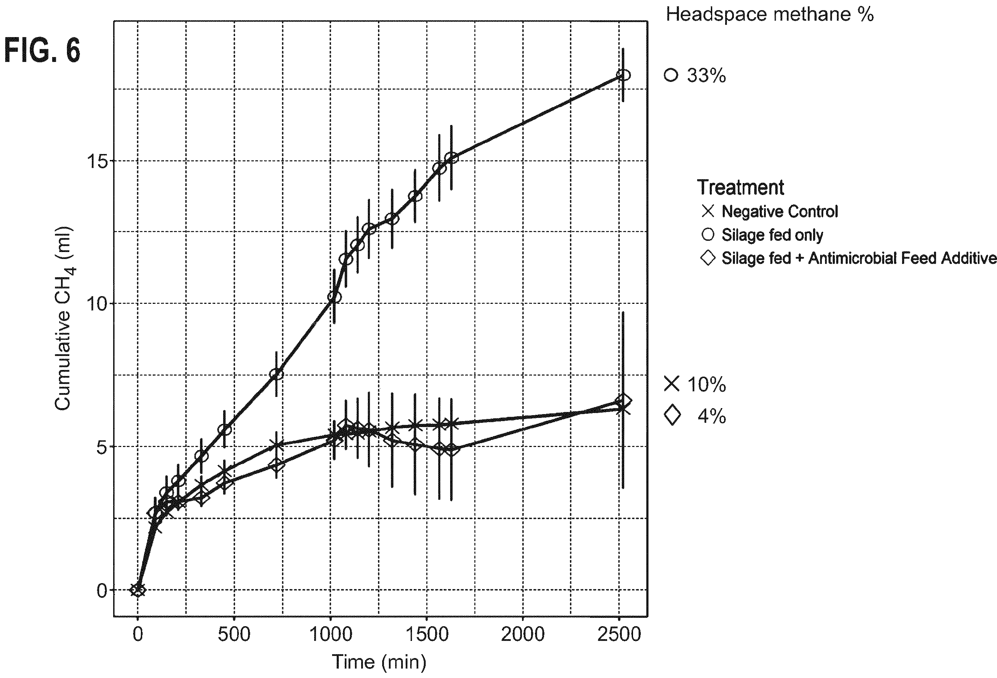

The results are shown in Figure 6.

### Example 5 - Protozoa counts

The procedures used in Example 4 were applied to rumen fluid, extracted from 6 slaughtered cows. Three cows were given the antimicrobial feed additive (comprising urea and potassium iodide) orally. Three cows were control, and were fed without a feed additive. Samples were taken and protozoa counts were taken.

Cows RF2, RF3 and RF5 were given the feed additive. RF1, RF4 and RF6 were fed without the feed additive.

### Protozoa counts

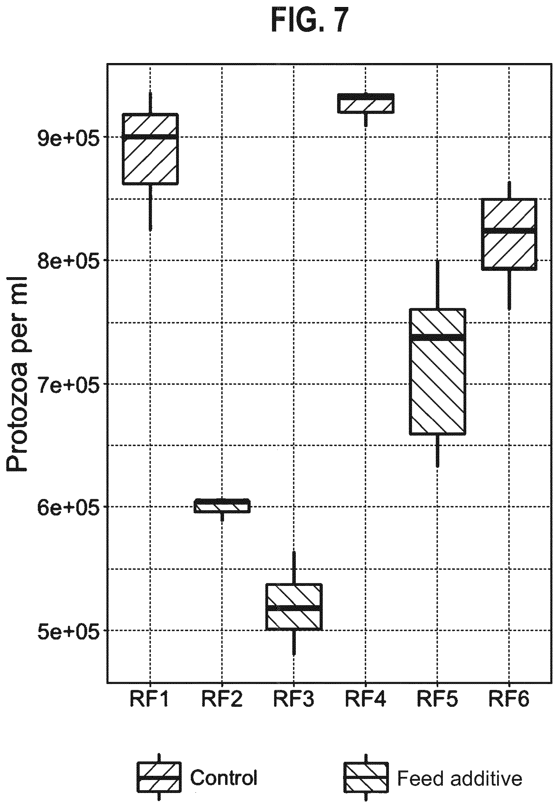

The results are shown in Figure 7.

These results show a clear reduction in the number of methanogens (in particular protozoa) in the rumen fluid from cows fed with the feed additive.

### Example 6 - Minimum inhibitory concentrations

### Background

In microbiology, the MIC is the lowest concentration of an antimicrobial that will inhibit the visible growth of a microorganism after overnight incubation (usually at 37 °C). MICs are important in diagnostic laboratories to confirm resistance of microorganisms to an antimicrobial agent and also to determine the potency of new antimicrobial agents.

### Broth microdilution method (MIC)

### Inoculum preparation:

1. Bacterial strains are grown overnight at 37°C, with shaking, in 20 ml LB broth.
2. The turbidity is adjusted with LB broth to gain a turbidity equivalent to the McFarland 0.5 standard. This is done spectrophotometrically. At 625 nm and using a 1 cm path, the desired absorbance will be 0.08-0.1. Once adjusted, the bacterial suspension will contain approximately 1 x 10⁸ cfu ml⁻¹.
3. 100 µl of the bacterial suspension is transferred to a tube containing 9.9 ml of LB.
   This results in an inoculum density of 1 x 10⁶ cfu ml⁻¹ which, when mixed with an equal volume of antimicrobial solution in wells, will result in a final inoculum of approximately 5 x 10⁵ cfu ml⁻¹.
4. 150 µl of the bacterial suspension is transferred to each well of columns 1-10 of a 96-well plate.
5. 150 µl of the bacterial suspension is transferred into column 11 of the 96-well plate.
   This will act as the positive control and will not receive any antimicrobial.
6. 150 µl of LB broth is transferred into column 12. This will not receive any bacteria or antimicrobial and will act as the negative control.

### Antimicrobial stock solutions preparation:

Stock solutions of Potassium Iodide (KI) in PBS (phosphate -buffered saline), and urea-hydrogen peroxide in PBS are prepared based on the desired ratio of FLChiKI (as specified in Tables 6 and 7 below). For example for a 3.5:1 ratio, a 1.6 mM KI solution and 25.2 mM H2O2 solution were used.

### Example microdilution (of a 3.5:1 ratio)

1. 75 µl of each stock solution were added to column one, starting with the KI.
2. The combined 300 µl bacterial suspension and antimicrobial in the well was mixed by pipetting up and down, giving a concentration of 350 mg H₂O₂/I.5 L: 100 mg KI/1.5 L. The concentration of the antimicrobial is based on the concentration of KI. Therefore, the first well contained 66.67 mg L 1 (or µg ml⁻¹) antimicrobial.
3. 150 µl of the suspension was transferred from column 1 into column 2 and mixed well. The concentration of antimicrobial in column two was therefore 33.33 mg L⁻¹.
4. Successive double dilutions were continued as far as column 10 (150 µl was discarded from wells in column 10 after mixing).
5. In this instance, concentrations were as follows: 66.67, 33.33, 16.67, 8.33, 4.17, 2.08, 1.04, 0.52, 0.26, 0.13 mg L⁻¹.

### Incubation of microdilution plate:

Incubate and monitor the growth of bacteria in a microtiter plate reader for 24 h at 37°C. Optical density measurements at 595 nm are recorded at 15 minute intervals. The cultures are agitated for 1 min after each measurement, allowing a 10 second rest before resumption of measurement.

Alternatively, the 96- well plate can be incubated at 37 °C for 24 h on a shaker.

### Determining MIC

MIC is determined by the lowest concentration of antimicrobial agent which showed less than a 0.1 optical density unit change relative to the negative control.

Alternatively, the results can be recorded based on visually assessing the turbidity in test wells by comparison to the negative control

### Results

MIC values at various ratios for H₂0₂:KI (by weight, as discussed above) were calculated, using the above protocol, for four common pathogens ( E . coli, P. aeruginosa, S. aureus, S. uberis).

**Table 6: MIC for four common pathogens based on KI**

| | | ***MIC in mgL⁻¹ based on KI*** | | | |
|---|---|---|---|---|---|
| **Ratio H₂O₂:KI** | **Amount H₂O₂:KI (mg)** | ***E. coli*** | ***P. aeruginosa*** | ***S. aureus*** | ***S. uberis*** |
| | | | | | |
| **10:1** | **3000:300** | 3.1-6.3 | 3.1-6.3 | 1.6-3.1 | 3.1-6.3 |
| | | | | | |
| **3.5:1** | **350:100** | 4.2-16.7 | 4.2-16.7 | 4.2-16.7 | 4.2-8.4 |
| | | | | | |
| **3:1** | **300:100** | 8.3-16.7 | 8.3-16.7 | 8.3-16.7 | 8.3-16.7 |
| **10:1** | **300:30** | 2.5-5 | 1.3-5 | 1.3-5 | 2.5-5 |
| **30:1** | **300:10** | 0.8-1.7 | 0.8-1.7 | 0.4-0.8 | 0.8-1.7 |
| | | | | | |
| **5:1** | **500:100** | 4.2-8.3 | 4.2-8.3 | 2.1-8.3 | 4.2-8.3 |
| **500:1** | **500:1** | 0.04-0.08 | 0.04-0.08 | 0.02-0.08 | 0.04-0.08 |
| | | | | | |
| **2:1** | **250:125** | 7.8-15.6 | | | |
| **4:1** | **250:62.5** | 3.9-7.8 | | | |
| **8:1** | **250:31.25** | 2.0-3.9 | | 3.9-7.8 | 3.9-7.8 |
| | | | | | |
| **2:1** | **100:50** | 16.7-33.3 | 16.7-33.3 | 8.3-16.7 | 16.7-33.3 |
| **10:3** | **100:30** | 10-20 | 10-20 | 5-10 | 10-20 |
| | | | | | |
| **3:1** | **30:10** | >6.7 | >6.7 | 3.3-6.7 | >6.7 |
| **5:1** | **150:30** | 5-10 | 5-10 | 2.5-5 | 5-10 |

**Table 7: MIC for four common pathogens based on I⁻**

| | | **MIC in mgL⁻¹ based on I⁻** | | | |
|---|---|---|---|---|---|
| **Ratio H₂O₂:I⁻¹** | **Amount H₂O₂:KI (mg)** | ***E. coli*** | ***P. aeruginosa*** | ***S. aureus*** | ***S. uberis*** |
| | | | | | |
| **13.1:1** | **3000:300** | 2.4-4.8 | 2.4-4.8 | 1.2-2.4 | 2.4-4.8 |
| | | | | | |
| **4.6:1** | **350:100** | 3.2-12.8 | 3.2-12.8 | 3.2-12.8 | 3.2-6.4 |
| | | | | | |
| **3.9:1** | **300:100** | 6.4-12.8 | 6.4-12.8 | 6.4-12.7 | 5.4-12.8 |
| **13.1:1** | **300:30** | 1.9-3.8 | 1.0-3.8 | 1.0-3.8 | 1.9-3.8 |
| **39.2:1** | **300:10** | 0.6-1.3 | 0.6-1.3 | 0.3-0.6 | 0.6-1.3 |
| | | | | | |
| **6.5:1** | **500:100** | 3.2-6.4 | 3.2-6.4 | 1.6-6.4 | 3.1-6.4 |
| **658:1** | **500:1** | 0.03-0.06 | 0.03-0.06 | 0.02-0.06 | 0.03-0.06 |
| | | | | | |
| **2.6:1** | **250:125** | 6.0-12.0 | | | |
| **5.2:1** | **250:62.5** | 3.0-6.0 | | | |
| **10.5:1** | **250:31.25** | 1.5-3.0 | | 3.0-6.0 | 3.0-6.0 |
| | | | | | |
| **2.6:1** | **100:50** | 12.7-25.3 | 12.7-25.3 | 6.3-12.7 | 12.7-25.3 |
| **4.4:1** | **100:30** | 7.6-15.2 | 7.6-15.2 | 3.8-7.6 | 7.6-15.2 |
| | | | | | |
| **3.9:1** | **30:10** | >5.1 | >5.1 | 2.5-5.1 | >5.1 |
| **6.6:1** | **150:30** | 3.8-7.6 | 3.8-7.6 | 1.9-7.6 | 3.8-7.6 |

### Discussion

Tables 6 and 7 show the same results - the numbers in the tables differ because Table 6 shows the results based on the amount of potassium iodide (i.e. KI), whereas Table 7 shows the results based on the amount of iodide ions (i.e. I⁻).

Surprisingly the compositions have very low MICs of less than, for example, 26 mg/L or 15 mg/L (based on I⁻) against a range of pathogens, i.e. it has been found that only a very low concentration of iodide ions is required in order to achieve antimicrobial activity. In fact, even a concentration of iodide ions of less than 5 mg/L was found to provide antimicrobial activity. Moreover, this high activity is obtained even when concentration of hydrogen peroxide is low. These compositions not only provide antimicrobial activity when used at low concentrations, but also provide the antimicrobial activity even if the ratio of iodide ions to hydrogen peroxide is low.

The results also show that even at a high ratio of hydrogen peroxide to iodide ions (e.g. of 653.6:1 or of 658:1), the composition provides very good antimicrobial activity. Indeed, the antimicrobial activity increased as the ratio of hydrogen peroxide to iodide ions increased. It is particularly surprising that this effect is seen when only a low concentration of hydrogen peroxide is used.

These impressive results were obtained when the composition was delivered in a saline solution. Thus, the compositions with low concentrations and the compositions with low iodide ratios of the invention are suitable for the treatment/prevention of microbial infections such as ocular infections, as they are effective at killing microorganisms, while being non toxic to animal or human tissue.

### Example 7 - Release profiles

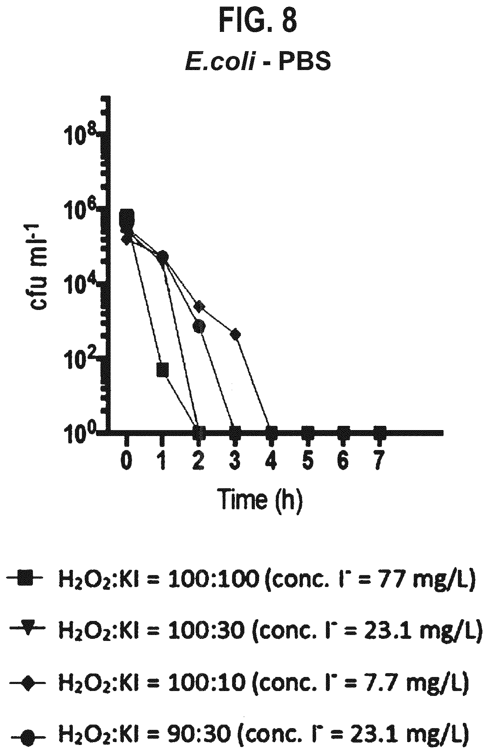

As can be seen from the data in Figure 8, the compositions with low concentrations of the invention were surprisingly found to provide excellent release profiles, i.e. the length of time it takes for the composition to start acting against the bacteria (e.g. E. coli ) inoculated in a sample is small. The release profiles were tested in phosphate buffered saline (PBS). The end result (at 4 hours) for the low concentration compositions of the invention and compositions with concentrations of, for example, 77 mg/L, was found to be the identical, which is surprising.

### Example 8 - low toxicity

### Summary

The biological balance between microbial and cellular toxicities can usefully be taken into consideration when contemplating the use of an antimicrobial composition for treatment of microbial infections. To assess the potential for cytotoxicity, the hemolytic activity of the compositions with low concentration and ratios of the invention were tested by determining the release of hemoglobin in horse erythrocyte suspension as a measure of red blood cell lysis *(*Evans et al., 2013, J. Vis. Exp. (73), e50166. doi: 10.3791/50166*).* A well-known antimicrobial agent, Lugol iodine, was included as a positive control.

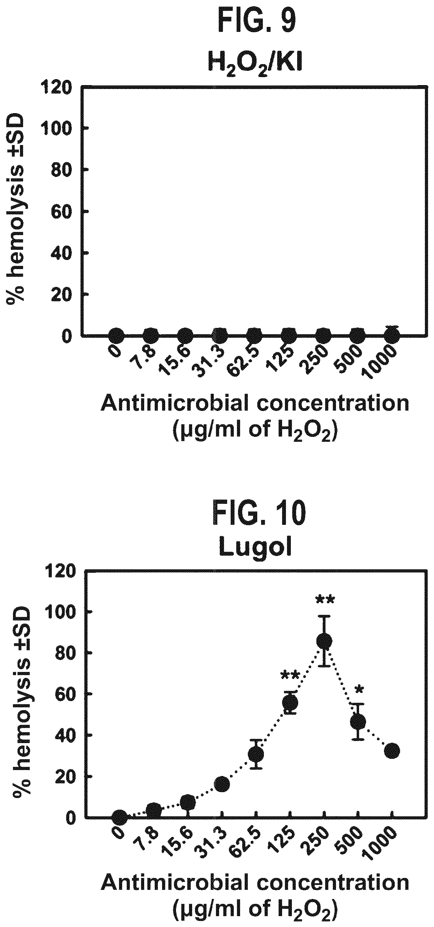

The results demonstrate that the compositions of low concentrations and ratios of the invention do not result in hemolytic activity after 1 hour of incubation with 4% horse red blood cells (Figures 9 and 10). Low toxicity to mammalian cells, while simultaneously having excellent antimicrobial effects, together with the possibility of delivering the low concentration/ratio compositions in a suitable diluent or carrier, such as a saline buffer indicates their suitability for use in the treatment and control of microbial infections, such as ocular infections.

### Method and results

Figures 9 and 10 show the hemolytic effect of the low concentration and low ratio compositions of the invention on horse red blood cells (final concentration 4%) as a function of antimicrobial concentration (0-1000 µg/ml of hydrogen peroxide, in a composition where the ratio of hydrogen peroxide to KI is 5: 1 - i.e. corresponding to 0-200 µg/ml KI) at 37°C after 1 h of incubation.

Lugol iodine was included as a positive antimicrobial control.

Each point in Figures 9 and 10 represent mean of % hemolysis of duplicate samples from two experiments, ±SD. One-way ANOVA followed by the Tukey's test was used for statistical analysis. * denotes to p-value <0.05, ** denotes to p-value <0.01 as compared with the untreated control. Insets are the representative assay plate wells containing the diluted supernatants of different treatments at various concentrations.

### Example 9 - Antibiotic combination: synergistic antimicrobial activity (illustrative to the claimed invention)

### Method

Checkerboard assays were carried out to determine if there is a synergistic antagonistic effect with the addition of an antibiotic to a composition comprising a source of iodide ions and a source of hydrogen peroxide.
- E. coli (1103 DSMZ) was used for tests
- These tests were carried out in lysogeny broth (lb)
- Antibiotics tested were ampicillin and gentamycin
- Procedure:
   - 50 µl antibiotic
   - 100 µl composition
   - 150 µl 105 cells in LB

### Results

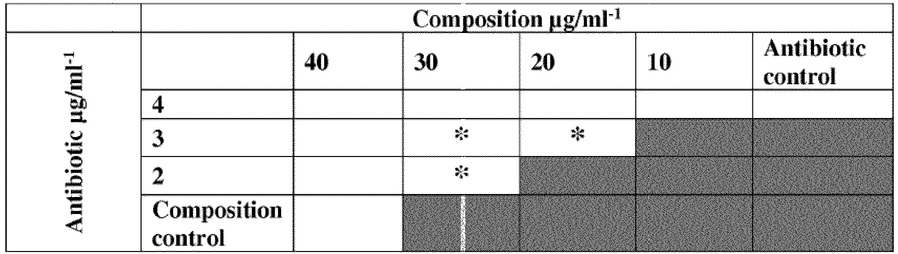

Cells shaded in dark grey represent sub-inhibitory concentrations where growth prevailed. Bacterial growth was prevented for the remaining cells. These results show that in the absence of the composition the concentration of antibiotic required to kill the bacteria was 4 µgml⁻¹ and the minimum concentration of the composition required to kill the bacteria was 40 µgml⁻¹. However, in the presence of both the composition and the antibiotic, the concentrations required to achieve a kill are lower - in other words, the combined MIC is lower. These can be seen by the entries marked with a star (*). The above results were seen for both antibiotics tested.

A synergistic effect equivalent to that shown above was also seen when erythromycin and clarithromycin were used as the antibiotic. Since the effect is observed from several antibiotics from several classes, the effect can therefore be expected with other antibiotics.

## Claims

1. Use of an antibiotic-free, antimicrobial feed additive in a method of improving the health of ruminant animals and reducing methane production of ruminant animals, wherein improving the health is:
(i) increasing milk production,
(ii) reducing finishing time,
(iii) increasing fertility, and
(iv) increasing production of volatile fatty acids,
wherein the feed additive comprises potassium iodide and urea, wherein the feed additive is administered orally,
wherein the method is not a method for treatment of the ruminant animal body by surgery or therapy,
wherein the potassium iodide is present in an amount of from 5-150 mg per gram of the feed additive and the urea is present in an amount of from 5-150 mg per gram of the feed additive, and wherein the feed additive does not contain one or more of the following vitamins: vitamin A, vitamin D and vitamin E.

2. The use according to claim 1, wherein the feed additive further comprises a source of hydrogen peroxide.

3. The use according to any preceding claim, wherein the feed additive does not contain a peroxidase enzyme.

4. The use according to any preceding claim, wherein the feed additive does not contain one or more of the following elements: chromium, cobalt, copper, manganese, selenium, magnesium and zinc.

5. The use according to any preceding claim, wherein the feed additive further comprises a cyanate compound such as potassium cyanate or thiocyanogens.

6. The use according to any one of claims 1 and 3-4, wherein the feed additive contains potassium iodide and urea as the sole active ingredients.

7. The use according to any preceding claim, wherein the feed additive is provided as a pellet.

8. The use according to any preceding claim, wherein the feed additive comprises food in combination with the potassium iodide and urea.

9. An antibiotic-free, antimicrobial feed additive, for use in a method of treating lameness in ruminant animals comprising orally administering the feed additive to the ruminant animals,
wherein the feed additive comprises potassium iodide and urea,
wherein the potassium iodide is present in an amount of from 5 -150 mg per gram of the feed additive and the urea is present in an amount of from 5 -150 mg per gram of the feed additive, and
wherein the feed additive does not contain one or more of the following vitamins: vitamin A, vitamin D and vitamin E.

10. An antibiotic-free, antimicrobial feed additive, for use in a method of treating acidosis in ruminant animals comprising orally administering the feed additive to the ruminant animals,
wherein the feed additive comprises potassium iodide and urea,
wherein the potassium iodide is present in an amount of from 5 -150 mg per gram of the feed additive and the urea is present in an amount of from 5-150 mg per gram of the feed additive, and
wherein the feed additive does not contain one or more of the following vitamins: vitamin A, vitamin D and vitamin E.

11. The feed additive for use according to claim 9 or 10, wherein the feed additive is provided as a pellet.

12. The feed additive for use according to any one of claims 9-11, wherein the feed additive comprises food in combination with the potassium iodide and urea.

13. A dosage form comprising potassium iodide and urea as the sole active ingredients, for use in a method of treating lameness in ruminant animals,
wherein the dosage form is orally administered,
wherein the potassium iodide is present in an amount of from 5 -150 mg per gram of the feed additive and the urea is present in an amount of from 5-150 mg per gram of the feed additive, and
wherein the feed additive does not contain one or more of the following vitamins: vitamin A, vitamin D and vitamin E.

14. A dosage form comprising potassium iodide and urea as the sole active ingredients, for use in a method of treating acidosis in ruminant animals,
wherein the dosage form is orally administered, wherein the potassium iodide is present in an amount of from 5-150 mg per gram of the feed additive and the urea is present in an amount of from 5 -150 mg per gram of the feed additive, and wherein the feed additive does not contain one or more of the following vitamins:
vitamin A, vitamin D and vitamin E.

## Patentansprüche

1. Verwendung eines antibiotikafreien, antimikrobiellen Futtermittelzusatzstoffs in einem Verfahren zum Verbessern der Gesundheit von Wiederkäuern und Reduzieren einer Methanproduktion von Wiederkäuern, wobei das Verbessern der Gesundheit Folgendes ist:
(i) Steigern einer Milchproduktion,
(ii) Reduzieren einer Fertigstellungszeit,
(iii) Steigern einer Fruchtbarkeit und
(iv) Steigern einer Produktion von flüchtigen Fettsäuren,
wobei der Futtermittelzusatzstoff Kaliumjodid und Harnstoff umfasst, wobei der Futtermittelzusatzstoff oral verabreicht wird,
wobei das Verfahren kein Verfahren zur Behandlung des Wiederkäuerkörpers durch Operation oder Therapie ist,
wobei das Kaliumjodid in einer Menge von 5-150 mg pro Gramm des Futtermittelzusatzstoffs vorhanden ist und der Harnstoff in einer Menge von 5-150 mg pro Gramm des Futtermittelzusatzstoffs vorhanden ist und wobei der Futtermittelzusatzstoff nicht eines oder mehrere der folgenden Vitamine enthält:
Vitamin A, Vitamin D und Vitamin E.

2. Verwendung nach Anspruch 1, wobei der Futtermittelzusatzstoff ferner eine Quelle von Wasserstoffperoxid umfasst.

3. Verwendung nach einem der vorhergehenden Ansprüche, wobei der Futtermittelzusatzstoff kein Peroxidase-Enzym enthält.

4. Verwendung nach einem der vorhergehenden Ansprüche, wobei der Futtermittelzusatzstoff eines oder mehrere der folgenden Elemente nicht enthält: Chrom, Kobalt, Kupfer, Mangan, Selen, Magnesium und Zink.

5. Verwendung nach einem der vorhergehenden Ansprüche, wobei der Futtermittelzusatzstoff ferner eine Cyanatverbindung wie Kaliumcyanat oder Thiocyanogene umfasst.

6. Verwendung nach einem der Ansprüche 1 und 3-4, wobei der Futtermittelzusatzstoff im Wesentlichen aus Kaliumjodid und Harnstoff besteht.

7. Verwendung nach einem der vorhergehenden Ansprüche, wobei der Futtermittelzusatzstoff als ein Pellet bereitgestellt ist.

8. Verwendung nach einem der vorhergehenden Ansprüche, wobei der Futtermittelzusatzstoff ein Lebensmittel in Kombination mit dem Kaliumjodid und Harnstoff umfasst.

9. Antibiotikafreier, antimikrobieller Futtermittelzusatzstoff zur Verwendung in einem Verfahren zum Behandeln von Lahmheit bei Wiederkäuern, umfassend ein orales Verabreichen des Futtermittelzusatzstoffs an die Wiederkäuer,
wobei der Futtermittelzusatzstoff Kaliumjodid und Harnstoff umfasst,
wobei das Kaliumjodid in einer Menge von 5-150 mg pro Gramm des Futtermittelzusatzstoffs vorhanden ist und der Harnstoff in einer Menge von 5-150 mg pro Gramm des Futtermittelzusatzstoffs vorhanden ist und
wobei der Futtermittelzusatzstoff nicht eines oder mehrere der folgenden Vitamine enthält: Vitamin A, Vitamin D und Vitamin E.

10. Antibiotikafreier, antimikrobieller Futtermittelzusatzstoff zur Verwendung in einem Verfahren zum Behandeln von Azidose bei Wiederkäuern, umfassend ein orales Verabreichen des Futtermittelzusatzstoffs an die Wiederkäuer,
wobei der Futtermittelzusatzstoff Kaliumjodid und Harnstoff umfasst,
wobei das Kaliumjodid in einer Menge von 5-150 mg pro Gramm des Futtermittelzusatzstoffs vorhanden ist und der Harnstoff in einer Menge von 5-150 mg pro Gramm des Futtermittelzusatzstoffs vorhanden ist und
wobei der Futtermittelzusatzstoff nicht eines oder mehrere der folgenden Vitamine enthält: Vitamin A, Vitamin D und Vitamin E.

11. Futtermittelzusatzstoff zur Verwendung nach Anspruch 9 oder 10, wobei der Futtermittelzusatzstoff als ein Pellet bereitgestellt ist.

12. Futtermittelzusatzstoff nach einem der Ansprüche 9-11, wobei der Futtermittelzusatzstoff ein Lebensmittel in Kombination mit dem Kaliumjodid und Harnstoff umfasst.

13. Darreichungsform, umfassend Kaliumjodid und Harnstoff als die einzigen Wirkstoffe, zur Verwendung in einem Verfahren zum Behandeln von Lahmheit bei Wiederkäuern, wobei die Darreichungsform oral verabreicht wird,
wobei das Kaliumjodid in einer Menge von 5-150 mg pro Gramm des Futtermittelzusatzstoffs vorhanden ist und der Harnstoff in einer Menge von 5-150 mg pro Gramm des Futtermittelzusatzstoffs vorhanden ist und
wobei der Futtermittelzusatzstoff nicht eines oder mehrere der folgenden Vitamine enthält:
Vitamin A, Vitamin D und Vitamin E.

14. Darreichungsform, umfassend Kaliumjodid und Harnstoff als die einzigen Wirkstoffe, zur Verwendung in einem Verfahren zum Behandeln von Azidose bei Wiederkäuern,
wobei die Darreichungsform oral verabreicht wird, wobei das Kaliumjodid in einer Menge von 5-150 mg pro Gramm des Futtermittelzusatzstoffs vorhanden ist und der Harnstoff in einer Menge von 5-150 mg pro Gramm des Futtermittelzusatzstoffs vorhanden ist und wobei der Futtermittelzusatzstoff nicht eines oder mehrere der folgenden Vitamine enthält:
Vitamin A, Vitamin D und Vitamin E.

## Revendications

1. Utilisation d'un additif alimentaire antimicrobien exempt d'antibiotique dans un procédé d'amélioration de la santé des animaux ruminants et de réduction de la production de méthane des animaux ruminants, dans laquelle l'amélioration de la santé consiste à :
(i) augmenter la production laitière,
(ii) réduire le temps de finition,
(iii) augmenter la fertilité, et
(iv) augmenter la production d'acides gras volatils, dans laquelle l'additif alimentaire comprend de l'iodure de potassium et de l'urée, dans laquelle l'additif alimentaire est administré par voie orale,
dans laquelle le procédé n'est pas un procédé de traitement du corps de l'animal ruminant par chirurgie ou thérapie,
dans laquelle l'iodure de potassium est présent en une quantité comprise entre 5 et 150 mg par gramme d'additif alimentaire et l'urée est présente en une quantité comprise entre 5 et 150 mg par gramme d'additif alimentaire, et dans laquelle l'additif alimentaire ne contient pas une ou plusieurs des vitamines suivantes :
vitamine A, vitamine D et vitamine E.

2. Utilisation selon la revendication 1, dans laquelle l'additif alimentaire comprend en outre une source de peroxyde d'hydrogène.

3. Utilisation selon une quelconque revendication précédente, dans laquelle l'additif alimentaire ne contient pas d'enzyme peroxydase.

4. Utilisation selon une quelconque revendication précédente, dans laquelle l'additif alimentaire ne contient pas un ou plusieurs des éléments suivants : chrome, cobalt, cuivre, manganèse, sélénium, magnésium et zinc.

5. Utilisation selon une quelconque revendication précédente, dans laquelle l'additif alimentaire comprend en outre un composé cyanate tel que le cyanate de potassium ou les thiocyanogènes.

6. Utilisation selon l'une quelconque des revendications 1 et 3 et 4, dans laquelle l'additif alimentaire est essentiellement composé d'iodure de potassium et d'urée.

7. Utilisation selon une quelconque revendication précédente, dans laquelle l'additif alimentaire est fourni sous forme de granulés.

8. Utilisation selon une quelconque revendication précédente, dans laquelle l'additif alimentaire comprend des aliments en combinaison avec de l'iodure de potassium et de l'urée.

9. Additif alimentaire antimicrobien exempt d'antibiotique, destiné à être utilisé dans un procédé de traitement de la boiterie chez les animaux ruminants comprenant l'administration par voie orale de l'additif alimentaire aux animaux ruminants,
dans lequel l'additif alimentaire comprend de l'iodure de potassium et de l'urée,
dans lequel l'iodure de potassium est présent en une quantité comprise entre 5 et 150 mg par gramme d'additif alimentaire et l'urée est présente en une quantité comprise entre 5 et 150 mg par gramme d'additif alimentaire, et
dans lequel l'additif alimentaire ne contient pas une ou plusieurs des vitamines suivantes : vitamine A, vitamine D et vitamine E.

10. Additif alimentaire antimicrobien exempt d'antibiotique, destiné à être utilisé dans un procédé de traitement de l'acidose chez les animaux ruminants comprenant l'administration par voie orale de l'additif alimentaire aux animaux ruminants,
dans lequel l'additif alimentaire comprend de l'iodure de potassium et de l'urée,
dans lequel l'iodure de potassium est présent en une quantité comprise entre 5 et 150 mg par gramme d'additif alimentaire et l'urée est présente en une quantité comprise entre 5 et 150 mg par gramme d'additif alimentaire, et
dans lequel l'additif alimentaire ne contient pas une ou plusieurs des vitamines suivantes : vitamine A, vitamine D et vitamine E.

11. Additif alimentaire destiné à être utilisé selon la revendication 9 ou 10, dans lequel l'additif alimentaire est fourni sous forme de granulés.

12. Additif alimentaire selon l'une quelconque des revendications 9 à 11, dans lequel l'additif alimentaire comprend des aliments en combinaison avec de l'iodure de potassium et de l'urée.

13. Forme posologique comprenant de l'iodure de potassium et de l'urée comme seuls ingrédients actifs, destinée à être utilisée dans un procédé de traitement de la boiterie chez les animaux ruminants, dans laquelle la forme posologique est administrée par voie orale,
dans lequel l'iodure de potassium est présent en une quantité comprise entre 5 et 150 mg par gramme d'additif alimentaire et l'urée est présente en une quantité comprise entre 5 et 150 mg par gramme d'additif alimentaire, et
dans lequel l'additif alimentaire ne contient pas une ou plusieurs des vitamines suivantes :
vitamine A, vitamine D et vitamine E.

14. Forme posologique comprenant de l'iodure de potassium et de l'urée comme seuls ingrédients actifs, destinée à être utilisée dans un procédé de traitement de l'acidose chez les animaux ruminants,
dans laquelle la forme posologique est administrée par voie orale, dans laquelle l'iodure de potassium est présent en une quantité comprise entre 5 et 150 mg par gramme d'additif alimentaire et l'urée est présente en une quantité comprise entre 5 et 150 mg par gramme d'additif alimentaire, et dans laquelle l'additif alimentaire ne contient pas une ou plusieurs des vitamines suivantes :
vitamine A, vitamine D et vitamine E.
